# NEUE EUROPÄISCHE PATENTSCHRIFT

(11) **EP 2 648 777 B2**
(45) Veröffentlichungstag und Bekanntmachung des Hinweises auf die Entscheidung über den Einspruch: **17.03.2021**
(45) Hinweis auf die Patenterteilung: 07.02.2018
(21) Anmeldenummer: 11794056.9
(22) Anmeldetag: 08.12.2011
(51) Int. Cl.: A61M 1/28, A61M 5/44, A61M 1/16

(54) **MEDIZINISCHES GERÄT MIT EINER HEIZUNG**
MEDICAL APPLIANCE WITH A HEATER
APPAREIL MÉDICAL ÉQUIPÉ D'UN CHAUFFAGE

(30) Priorität: 09.12.2010 DE 102010053973; 09.12.2010 US 421332 P
(43) Veröffentlichungstag der Anmeldung: 16.10.2013
(73) Patentinhaber: Fresenius Medical Care Deutschland GmbH, 61352 Bad Homburg (DE)
(72) Erfinder: HEDMANN, Frank, 97332 Volkach (DE); SEBESTA, Sven, 97421 Schweinfurt (DE); WERNICKE, Ulrich, 97531 Theres (DE)
(74) Vertreter: Laufhütte, Dieter
(86) Internationale Anmeldenummer: PCT/EP2011/006188
(87) Internationale Veröffentlichungsnummer: WO 2012/076179

(56) Entgegenhaltungen:
- EP-A2- 0 380 140
- EP-A2- 0 804 049
- EP-A2- 1 894 586
- WO-A2-2009/105413
- DE-A1- 2 618 415
- US-A- 4 923 681
- US-A1- 2009 312 694
- H.-U. GIERSCH et al.: "Elektrotechnik für Fachschulen" In: 1998, Springer pages 10-103,
- WILFRIED PLAßMANN et al.: In: "Handbuch Elektrotechnik - Grundlagen und Anwendungen für Elektrotechniker", 2009 pages 319-459,
- E. BÖHMER et al.: "Thyristoren und Triacs" In: "Elemente der angewandten Elektronik - Kompendium für Ausbildung und Beruf", 2007 pages 306-321,

## Beschreibung

Die vorliegende Erfindung betrifft ein medizinisches Gerät mit einer Heizung mit mindestens einem Heizelement, welches von einer Heizungssteuerung mit Netzspannung beaufschlagt wird. Insbesondere handelt es sich dabei um ein Dialysegerät mit einer Heizung zur Erwärmung von medizinischen Flüssigkeiten, insbesondere um ein Peritonealdialysegerät mit einer Heizung zur Erwärmung des Dialysats.

Bei einem solchen medizinischen Gerät ist die Heizung üblicherweise als ein Ohmsches Heizelement realisiert, an welches die Heizungssteuerung Netzspannung anlegt, um das Heizelement einzuschalten bzw. das Heizelement von der Netzspannung trennt, um dieses auszuschalten.

Zur Einstellung der Heizleistung und zur Anpassung an unterschiedliche Nennspannungen ist es bereits bekannt, die Heizung in mehrere Heizelemente aufzuteilen oder die Heizelemente über eine Phasenanschnittssteuerung anzusteuern.

Phasenanschnittssteuerungen sind jedoch kompliziert und haben zudem Probleme mit elektromagnetischer Abstrahlung. Zudem besteht hier eine erhebliche Verlustleistung in der Elektronik. Die bisher bekannte Aufteilung in mehrere Heizelemente hat weiterhin den Nachteil, daß die Geräte bei unterschiedlichen Nennspannungen der Netzspannung unterschiedlich geschaltet werden müssen, um keine unzulässig hohen Stromaufnahmen zu erreichen.

Aus der WO 2009/105413 A2 ist weiterhin ein Dialysegerät mit einer Heizungssteuerung bekannt, welche die Netzwechselspannung jeweils im Nulldurchgang schaltet, um Strahlungsemissionen zu verringern. Dabei wird zur Steuerung der Heizung die Netzwechselspannung mit einer im Vergleich zur Frequenz der Netzwechselspannung niedrigen Frequenz pulsweitenmoduliert, so dass die Heizelemente jeweils für eine Vielzahl von Zyklen eingeschaltet und ausgeschaltet werden.

Die aus der WO 2009/105413 A2 bekannte Heizung weist dabei zwei Heizelemente auf und überwacht die Höhe der Netzwechselspannung. Zur Anpassung der Leistung der Heizung an die Höhe der Netzwechselspannung werden entweder beide Heizelemente parallel betrieben oder nur eines der Heizelemente. In einem weiteren Ausführungsbeispiel werden die Heizelemente entweder parallel oder seriell betrieben.

Aufgabe der vorliegenden Erfindung ist es, ein medizinisches Gerät mit einer Heizung zur Verfügung zu stellen, welche eine verbesserte Heizungssteuerung aufweist.

Diese Aufgabe wird erfindungsgemäß durch ein medizinisches Gerät gemäß Anspruch 1 gelöst. Das medizinische Gerät der vorliegenden Erfindung weist dabei eine Heizung mit mindestens einem Heizelement und mit einem Temperatursensor sowie eine Heizungssteuerung auf, wobei die Heizungssteuerung das Heizelement mit Netzspannung beaufschlagt. Erfindungsgemäß umfaßt die Heizungssteuerung dabei eine Überwachungsanordnung und eine Schaltanordnung, wobei die Überwachungsanordnung die Nulldurchgänge der Netzspannung erkennen und die Schaltanordnung das mindestens eine Heizelement im Nulldurchgang ein- oder ausschalten kann, wobei die Heizungssteuerung die Leistung der Heizung über das Ein- und Ausschalten einer oder mehrerer Halbwellen der Netzspannung ansteuert und wobei das Verhältnis der Anzahl der Halbwellen mit eingeschalteten Heizelement zur Anzahl der Halbwellen mit ausgeschalteten Heizelement in Abhängigkeit von einem Signal des Temperatursensors eingestellt wird. Die vorliegende Erfindung stellt so eine äußerst einfache und effektive Heizungssteuerung zur Verfügung. Insbesondere können dabei erfindungsgemäß einzelne Halbwellen der Netzspannung ein- bzw. ausgeschaltet werden. Selbstverständlich können jedoch auch Pulspakete mit mehreren Halbwellen oder Perioden der Netzspannung geschaltet werden. Vorteilhafterweise wird dabei die Leistung über das Verhältnis der Anzahl der Halbwellen mit eingeschaltetem Heizelement zur Anzahl der Halbwellen mit ausgeschaltetem Heizelement eingestellt. Gegenüber einer Phasenanschnittssteuerung wird dabei die Abstrahlung, die Anzahl der Bauteile und die Verlustleistung in der Elektronik deutlich verringert.

Gemäss der vorliegenden Erfindung detektiert die Überwachungsanordnung weiterhin die Höhe der Netzspannungsversorgung, wobei die Heizungssteuerung die Ansteuerung des mindestens einen Heizelements an die detektierte Höhe der Netzspannungsversorgung anpaßt. Hierdurch kann das erfindungsgemäße medizinische Gerät mit unterschiedlichen Nennspannungen der Netzspannung betrieben werden. Insbesondere ermöglicht die erfindungsgemäße Steuerung der Leistung der Heizung über das Ein- und Ausschalten einer oder mehrerer Halbwellen der Netzspannung einen Betrieb mit unterschiedlichen Nennspannungen der Netzspannungsversorgung und/oder eine Anpassung an schwankende Spannungspegel der Netzspannungsversorgung. Insbesondere kann so die gleiche Maximalleistung der Heizung bei unterschiedlichen Netzwechselspannungen bereitgestellt werden. Insbesondere wird dabei das Verhältnis der Anzahl der Halbwellen mit eingeschaltetem Heizelement zur Anzahl der Halbwellen mit ausgeschaltetem Heizelement an die detektierte Höhe der Netzspannungsversorgung angepaßt, insbesondere so, dass unabhängig von der Höhe der Netzspannungsversorgung jeweils die gleiche Maximalleistung der Heizung zur Verfügung steht.

Das erfindungsgemäße medizinische Gerät kann weiterhin mindestens zwei Heizelemente aufweisen, welche von der Schaltanordnung unabhängig voneinander ein- und ausgeschaltet werden können. Die Aufteilung in zwei Heizelemente erlaubt dabei eine noch flexiblere Ansteuerung der Leistung der Heizung.

Hinsichtlich der oben beschriebenen Ansteuerung der Leistung der Heizung über die Anzahl der Halbwellen mit ein- bzw. ausgeschaltetem Heizelement wird dabei vorteilhafterweise die Anzahl der Halbwellen, bei welchen das erste Heizelement eingeschaltet ist, und die Anzahl der Halbwellen, bei welchen das zweite Heizelement eingeschaltet ist, addiert, gegebenenfalls unter Berücksichtigung eines Faktors zur Berücksichtigung unterschiedlicher Nennleistungen der beiden Heizelemente. Gleiches gilt für die Anzahl der Halbwellen mit jeweils ausgeschaltetem ersten bzw. zweiten Heizelement.

In vorteilhafter Weise weist die Heizungssteuerung dabei ein ersten Betriebsmodus auf, in welchem die beiden Heizelemente teilweise oder durchgehend synchron betrieben werden. Insbesondere werden die beiden Heizelemente dabei teilweise oder durchgehend synchron mit Netzspannungshalbwellen beaufschlagt. Durch die synchrone Betriebesweise der beiden Heizelemente läßt sich eine entsprechend hohe Leistung auch bei einer niedrigen Versorgungsspannung erzielen. Zur Verringerung der Leistung können dabei beide Heizelemente für eine entsprechende Anzahl von Halbwellen synchron abgeschaltet werden. Alternativ ist es jedoch auch denkbar, zur Verringerung der Leistung jeweils nur eines der beiden Heizelemente abzuschalten.

Weiterhin vorteilhafterweise weist die erfindungsgemäße Heizungssteuerung einen zweiten Betriebsmodus auf, in welchem die mindestens zwei Heizelemente abwechselnd betrieben werden. Insbesondere werden die beiden Heizelemente dabei abwechselnd mit einer bestimmten Anzahl von Netzspannungshalbwellen beaufschlagt. Insbesondere kann in diesem zweiten Betriebsmodus vorgesehen sein, daß für alle Netzspannungshalbwellen, in welchen das erste Heizelement eingeschaltet ist, das zweite Heizelement ausgeschaltet ist und umgekehrt. Selbstverständlich können bei diesem abwechselnden Betrieb auch beide Heizelemente ausgeschaltet sein. Der abwechselnde Betrieb der zwei Heizelemente, insbesondere der Betrieb der zwei Heizelemente mit jeweils einer Halbwelle, ermöglicht es, die Stromstärken und/oder Leistung auch bei hohen Versorgungsspannungen in einem zulässigen Bereich zu halten. Insbesondere werden die beiden Heizelemente dabei jeweils abwechselnd mit aufeinanderfolgenden Halbwellen betrieben.

In weiterhin vorteilhafter Weise ist dabei vorgesehen, daß die Heizungssteuerung in Abhängigkeit von der detektierten Höhe der Netzspannungsversorgung den ersten oder zweiten Betriebsmodus auswählt. Insbesondere kann so sichergestellt werden, daß trotz unterschiedlichen Nennspannungen der Netzspannungsversorgung die gleiche maximale Heizleistung zur Verfügung steht. Weiterhin können durch den zweiten Betriebsmodus auch bei hohen Nennspannungen Ströme vermieden werden, welche die Netzspannungsversorgung und/oder die Heizelemente überlasten würden.

In vorteilhafter Weise wählt die Heizungssteuerung dabei bei Detektion einer Netzwechselspannung, welche sich in einem ersten, niedrigeren Spannungsbereich befindet, den ersten Betriebsmodus und bei Detektion einer Netzwechselspannung, welche sich in einem zweiten, höheren Spannungsbereich befindet, den zweiten Betriebsmodus aus. In vorteilhafter Weise umfasst der erste, niedrigere Bereich dabei zumindest eine Netzwechselspannung zwischen 100 V und 120 V, insbesondere 100 V, 110 V oder 120 V. In weiterhin vorteilhafter Weise umfasst der zweite, höhere Bereich zumindest eine Netzwechselspannung zwischen 230 V und 250 V, insbesondere 230 V oder 240 V. Insbesondere umfasst der erste Bereich dabei den Bereich zwischen 90 V und 110 V, weiterhin vorteilhafterweise zwischen 80 V und 130 V, weiterhin vorteilhafterweise zwischen 80 V und 160 V. Weiterhin vorteilhafterweise umfasst der zweite Bereich dabei den Bereich zwischen 200 V und 240 V, weiterhin vorteilhafterweise zwischen 180 V und 250 V, weiterhin vorteilhafterweise zwischen 160 V und 250 V.

Vorteilhafterweise wird bei einem Betrieb im ersten und/oder im zweiten Betriebsmodus das Verhältnis der Anzahl der Halbwellen mit eingeschalteten Heizelementen zur Anzahl der Halbwellen mit ausgeschalteten Heizelementen in Abhängigkeit von der Höhe der detektierten Netzwechselspannung eingestellt. So kann bei einer Netzwechselspannung, welche innerhalb des jeweiligen Spannungsbereiches, in welchem ein Betrieb im ersten und/oder im zweiten Betriebsmodus erfolgt, die Maximalleistung der Heizung konstant gehalten und/oder eine gewünschte Leistung eingestellt werden.

Insbesondere kann das medizinische Gerät dabei im ersten Betriebsmodus so betrieben werden, daß die zwei Heizelemente zur Einstellung der Leistung nicht mit sämtlichen Netzspannungshalbwellen beaufschlagt, sondern für ein oder mehrere Netzspannungshalbwellen synchron oder abwechselnd ein- und ausgeschaltet werden.

Im zweiten Betriebsmodus wird nicht jede Halbwelle entweder auf das eine oder andere Heizelement geschaltet, sondern es werden zur Reduktion der Leistung eine entsprechende Anzahl von Halbwellen auf keines der Heizelemente geschaltet. Die Anzahl der Netzspannungshalbwellen, mit welchen die Heizelemente dabei beaufschlagt werden, kann dabei in Abhängigkeit von der Höhe der Netzwechselspannung entsprechend verändert werden.

Selbstverständlich kann die vorliegende Erfindung auch mit mehr als nur zwei Heizelementen eingesetzt werden. Zum Beispiel könnte die vorliegende Erfindung mit drei oder vier Heizelementen implementiert werden, wobei dann im zweiten Betriebsmodus die drei oder vier Heizelemente jeweils abwechselnd mit Halbwellen beaufschlagt werden, d.h. so, daß eine Halbwelle immer maximal auf eines der Heizelemente geschaltet wird. Der abwechselnde Betrieb kann dabei zum Beispiel erfolgen, indem die eine oder mehreren Halbwellen den einzelnen Heizelementen nacheinander aufgeschaltet werden. Hierdurch kann der Heizungswiderstand jedes einzelnen Heizelements erhöht und so die maximale Stromaufnahme bei hoher Netzspannung entsprechend reduziert werden. Bei niedriger Spannung können die Heizelemente dann im ersten Betriebsmodus parallel betrieben werden. Selbstverständlich sind dabei auch mehr als vier Heizelemente denkbar.

Bei den oben beschriebenen bevorzugten Ausführungsbeispielen der vorliegenden Erfindung wurde die Leistung der Heizung über das Ein- und Ausschalten einer oder mehrerer Halbwellen der Netzspannung an unterschiedliche Höhen der Netzspannungsversorgung angepaßt. Insbesondere kann so die Maximalleistung der Heizung für unterschiedliche Netzspannungen gleich gehalten werden. Zudem kann das Auftreten unzulässig hoher Ströme verhindert werden.

Selbstverständlich kann die vorliegende Erfindung jedoch auch dazu eingesetzt werden, um die Leistung der Heizung zu Zwecken der Temperaturregelung auf einen Wert unterhalb der Maximalleistung einzustellen. Insbesondere kann die vorliegende Erfindung dazu genutzt werden, die Heizung auf einen Wert zwischen 0 und 100% der Maximalleistung einzustellen. Auch hier kann die aktuell abgegebene Leistung durch das Verhältnis der Anzahl der Halbwellen mit eingeschaltetem Heizelement zur Anzahl der Halbwellen mit ausgeschaltetem Heizelement eingestellt werden.

In vorteilhafter Weise umfaßt das medizinische Gerät dabei einen Temperatursensor, wobei das Verhältnis der Anzahl der Halbwellen mit eingeschaltetem Heizelement zur Anzahl der Halbwellen mit ausgeschaltetem Heizelement in Abhängigkeit von einem Signal des Temperatursensors eingestellt wird.

Eine solche Temperaturregelung kann dabei auch unabhängig von der Anpassung der Leistung der Heizung an unterschiedliche Netzspannungen eingesetzt werden, insbesondere auch bei solchen Geräten, welche nur mit einer einzigen Netzspannung einsetzbar sind. In vorteilhafter Weise wird eine solche Temperaturregelung jedoch mit einer Anpassung an die Betriebsspannung der Netzspannungsversorgung kombiniert.

Im einem bevorzugten Ausführungsbeispiel erzeugt die Heizungssteuerung daher auf Grundlage des Signals des Temperatursensors ein Steuersignal, welches den Steuersignalen zur Anpassung der Leistung an die detektierte Höhe der Netzspannungsversorgung überlagert wird. Für eine solche Überlagerung sind unterschiedliche Ausführungen denkbar.

Insbesondere kann anhand des Signals des Temperatursensors ein Hüllsignal mit einer im Vergleich zur Netzspannungsperiode längeren Schaltdauer erzeugt werden, welches der mit einer oder mehreren Netzspannungshalbwellen arbeitenden Anpassung der Leistung an die detektierte Höhe der Netzspannungsversorgung überlagert wird. Alternativ kann das Verhältnis der Anzahl der Halbwellen mit eingeschaltetem Heizelement zur Anzahl der Halbwellen mit ausgeschaltetem Heizelement direkt in Abhängigkeit von dem Signal des Temperatursensors und der detektierten Höhe der Netzspannungsversorgung zeitlich gleichmäßig eingestellt werden.

Bei der vorliegenden Erfindung werden erfindungsgemäß eine oder mehrere Halbwellen der Netzspannung ein- bzw. ausgeschaltet. In einer ersten Ausführungsform können dabei einzelne Netzspannungshalbwellen ein- und ausgeschaltet werden. Es können jedoch auch Pulspakete aus mehreren Halbwellen der Netzspannung ein- und ausgeschaltet werden, beispielsweise Pulspakete aus 1 bis 100 Netzspannungshalbwellen, weiterhin in vorteilhafter Weise aus 1 bis 10 Netzspannungshalbwellen.

Das Heizelement ist dabei in seiner Reaktion relativ träge, so daß die Temperatur des Heizelements auch bei der Verwendung mehrerer Halbwellen der Netzspannung sich nicht mit dem Ein- und Ausschalten der Halbwellen relevant erhöht und erniedrigt, sondern nur über das mittlere Verhältnis der Anzahl von ein- und ausgeschalteten Halbwellen bestimmt wird. Um eine möglichst feinkörnige Einstellung der Leistung über einen großen Leistungsbereich und/oder einen großen Bereich von Netzwechselspannungen zu erreichen, sollte die zur Ansteuerung verwendete kleinste Anzahl von schaltbaren Halbwellen jedoch vorteilhafterweise relativ niedrig gehalten werden, insbesondere bei 1 bis 5, weiterhin bei 1 bis 3 Halbwellen.

Das Verhältnis der Anzahl der Halbwellen mit eingeschaltetem Heizelement zur Anzahl der Halbwellen mit ausgeschaltetem Heizelement wird dabei in vorteilhafter Weise für einen bestimmten Zeitraum bzw. eine bestimmte Anzahl von Halbwellen bestimmt und zur Ansteuerung herangezogen. Ein typischer Zeitraum kann dabei beispielsweise zwischen 0,1 und 20 Sekunden liegen, vorteilhafterweise zwischen 0,5 und 5 Sekunden.

Z.B. kann die Heizungssteuerung dabei zu jedem Zeitpunkt die jeweils nächsten Halbwellen so ein- oder ausschalten, daß das Verhältnis innerhalb des zur Bestimmung herangezogenen Zeitraums auf einem Sollwert bleibt.

Ebenso ist es denkbar, das für die Bereitstellung der gewünschten Leistung notwenige Verhältnis der Anzahl der Halbwellen mit eingeschaltetem Heizelement zur Anzahl der Halbwellen mit ausgeschaltetem Heizelement jeweils nach einem festen Zeitraum oder einer festen Anzahl von Halbwellen neu zu bestimmen und dann im darauffolgenden Zeitraum bzw. bei der darauffolgenden festen Anzahl von Halbwellen eine entsprechende Ansteuerung vorzunehmen. Ein typischer Zeitraum kann dabei wiederum zwischen 0,1 und 20 Sekunden liegen, vorteilhafterweise zwischen 0,5 und 5 Sekunden. Insbesondere wird dabei das Verhältnis jeweils neu aufgrund der gemessenen Netzwechselspannung und der gewünschten Leistung berechnet. Die vorliegende Erfindung kann insbesondere bei einem Dialysegerät zum Einsatz kommen, wobei die Heizung zur Erwärmung einer medizinischen Flüssigkeit, insbesondere zur Erwärmung von Dialysat oder Blut, eingesetzt wird. In einer besonders vorteilhaften Ausgestaltung der vorliegenden Erfindung handelt es sich bei dem medizinischen Gerät dabei um ein Peritonealdialysegerät mit einer Heizung zur Erwärmung des Dialysats. Die vorliegende Erfindung kann ebenso bei Infusionsgeräten eingesetzt werden, insbesondere zur Erwärmung einer Infusionslösung. Die vorliegende Erfindung kann dabei mit beliebigen Ausführungen einer solchen Heizung zur Erwärmung des Dialysats eingesetzt werden, insbesondere bei einer Durchflußheizung, einer Heizung mittels eines Heizbeutels oder einer Heizung zur Erwärmung der Vorlagebeutel. Als Temperatursensor zur Regelung der Temperatur kann dabei ein Temperatursensor eingesetzt werden, welcher die Temperatur des Heizelementes direkt mißt. Alternativ oder zusätzlich kann auch ein Temperatursensor eingesetzt werden, welcher die Temperatur des zur erwärmenden Mediums bestimmt, insbesondere die Temperatur des Dialysats beim Einsatz in ein Peritonealdialysegerät.

Neben dem medizinischen Gerät mit einer erfindungsgemäßen Heizung offenbart die vorliegende Erfindung weiterhin eine Heizungssteuerung für ein medizinisches Gerät, wie es oben beschrieben wurde. Eine solche Heizungssteuerung hat dabei die Vorteile, wie sie bereits oben beschrieben wurden.
Die vorliegende Erfindung umfaßt weiterhin ein Verfahren zum Betrieb eines medizinischen Gerätes mit einer Heizung mit mindestens einem Heizelement oder zum Betrieb einer Heizungssteuerung für ein solches Gerät, mit den Schritten: Erfassen der Nulldurchgänge der Netzspannung und Ein- und Ausschalten des mindestens einen Heizelements im Nulldurchgang, wobei die Leistung der Heizung über die Anzahl der Halbwellen der Netzspannung mit eingeschaltetem Heizelement angesteuert wird. Das Verfahren erfolgt dabei so, wie dies oben bezüglich des medizinischen Gerätes näher dargestellt wurde. Insbesondere handelt es sich dabei bei dem erfindungsgemäßen Verfahren um ein Verfahren zum Betrieb eines medizinischen Gerätes oder einer Heizungssteuerung, wie sie oben beschrieben wurden.
Die vorliegende Erfindung soll nun anhand von Ausführungsbeispielen sowie Zeichnungen näher dargestellt werden.
Dabei zeigen:
- Fig. 1: drei Diagramme, welche typische Verläufe einer automatischen Peritonealdialysebehandlung zeigen,
- Fig. 2: eine Prinzipdarstellung eines Peritonealdialysesystems,
- Fig. 3: eine Prinzipdarstellung der Aufteilung des Peritonealdialysesystems in eine Dialysemaschine und ein Fluidsystem,
- Fig. 4: ein erstes Ausführungsbeispiel einer Kassette,
- Fig. 5: ein zweites Ausführungsbeispiel einer Kassette,
- Fig. 6: eine perspektivische Ansicht eines ersten Ausführungsbeispiels einer Dialysemaschine,
- Fig. 7: ein Flussdiagramm eines ersten Ausführungsbeispiels eines Peritonealdialysesystems,
- Fig. 8: eine perspektivische Ansicht eines zweiten Ausführungsbeispiels einer Dialysemaschine,
- Fig. 9: ein Flussdiagramm eines zweiten Ausführungsbeispiels eines Peritonealdialysesystems,
- Fig. 10: das Ankoppeln der Kassette beim zweiten Ausführungsbeispiels eines Peritonealdialysesystems,
- Fig. 11: ein erstes Ausführungsbeispiels eines Pumpaktors,
- Fig. 12: das Ankoppeln eines Pumpbereichs der Kassette an einen Pumpaktor,
- Fig. 13: eine Prinzipdarstellung des Aufbaus eines Ausführungsbeispiels einer Steuerung,
- Fig. 14: eine Prinzipdarstellung eines Ausführungsbeispiels einer erfindungsgemäßen Heizung und
- Fig. 15a und 15b: zwei Diagramme, welche die an den zwei Heizelementen des in Fig. 14 gezeigten Ausführungsbeispiels anliegenden netzspannungshalbwellen in zwei verschiedenen Betriebsmodi zeigen.

Im folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritoneum) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d.h. während der Patient schläft.

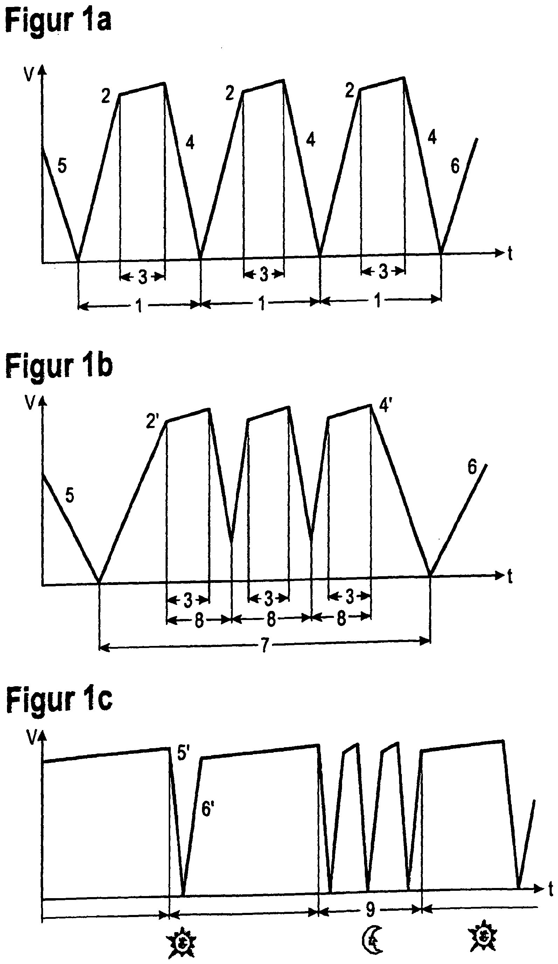

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1 a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1 a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 l. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewissen Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1 a oder 1b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

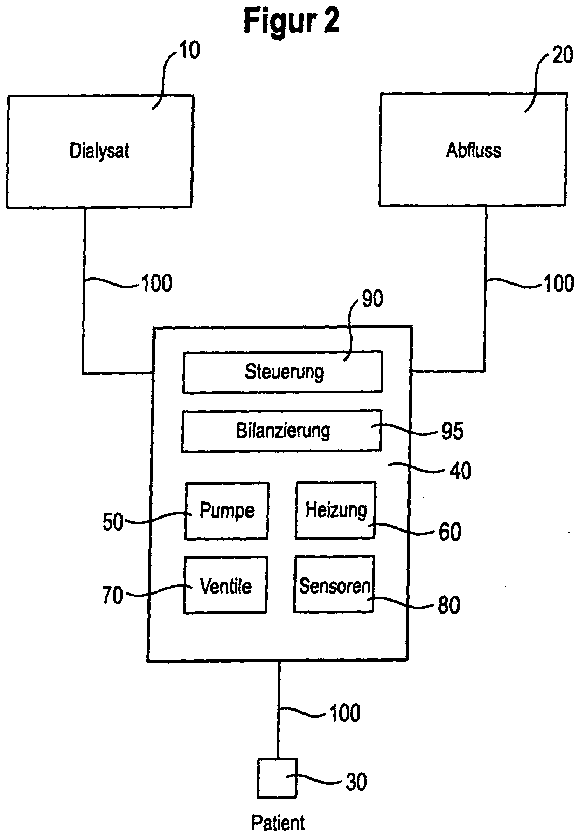

In Figur 2 ist nun der Aufbau eines typischen Peritonealdialysesystems schematisch dargestellt. Das Peritonealdialysesystem umfasst dabei einen Behälter 10 mit frischem Dialysat und einen Abfluss 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluss 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfasst dabei folgende Hauptkomponenten:
- Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluss 20 transportieren.
- Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluss 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an. Die Steuerung 90 sorgt dabei für den automatischen Ablauf der Peritonealdialyse. Als wichtige Komponente umfasst die Steuerung 90 dabei eine Bilanzierung 95, welche die dem Patienten zugegebenen und entnommenen Flüssigkeitsmengen bilanziert. Die Bilanzierung verhindert dabei, dass dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

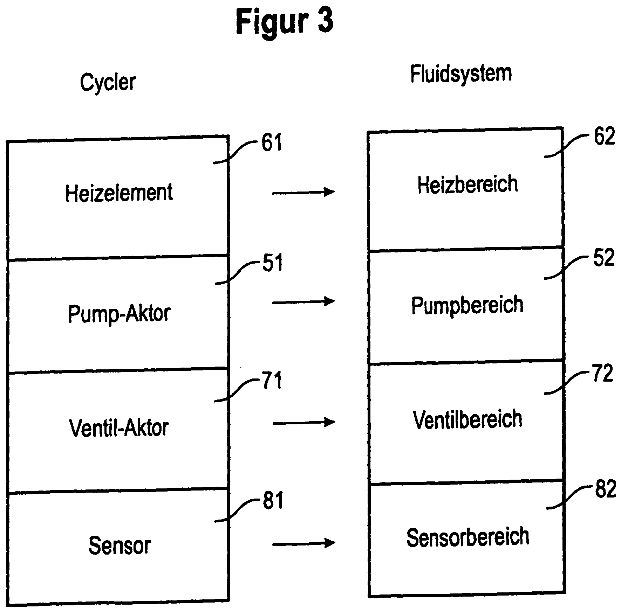

Um dennoch eine Steuerung der Peritonealdialyse durch die Dialysemaschine 40 zu ermöglichen, muß das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muss an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereiche 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, dass der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststofffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, dass das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfasst dabei üblicherweise den Puffer, während eine zweite Einzellösung Glucose und Elektrolyte umfasst. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventilen zum Befüllen des Patienten herangezogen werden.

### 1.2 Abfluss

Zum Entsorgen der verbrauchten Dialysierflüssigkeit kann diese entweder sofort in die Kanalisation abgeführt oder zunächst in einem Abflussbehälter gesammelt werden. Als Abflussbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muss. Hierfür muss das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in welchen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststofffolie bedeckt, welche für die Ankopplung an die Dialysemaschine sorgt. Die flexible Kunststofffolie ist dabei üblicherweise in einem Randbereich mit dem Hartteil verschweißt. Die Kassette wird mit einer Ankoppelfläche der Dialysemaschine verpresst, so dass die Aktoren und/oder Sensoren der Dialysemaschine mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfasst dabei üblicherweise zumindest einen Pumpbereich und einen oder mehrere Ventilbereiche. Über die Kassette kann so der Flüssigkeitstransport durch das Fluidsystem gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

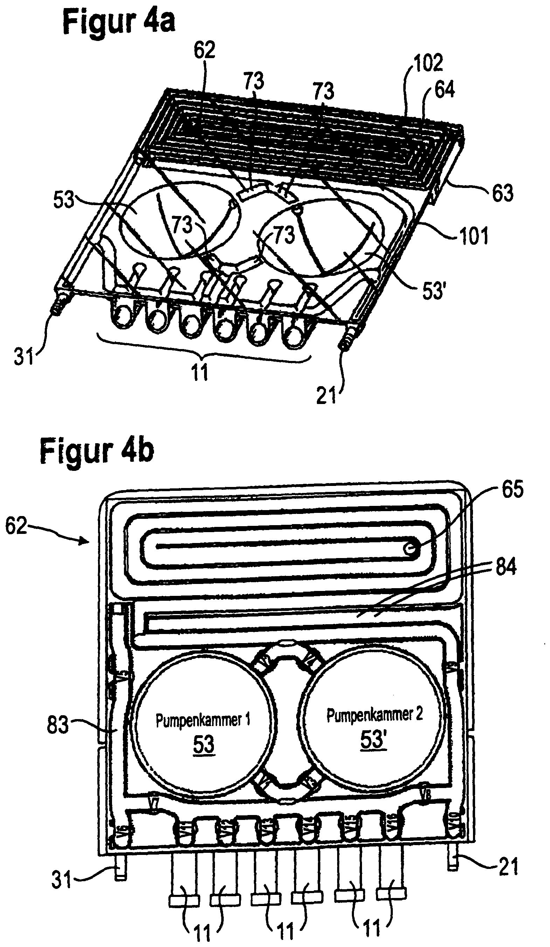

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil gefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpresst. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum einen ist ein Anschluss 21 zum Anschluss an den Abfluss 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, dass der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluss 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können.

Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschienenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

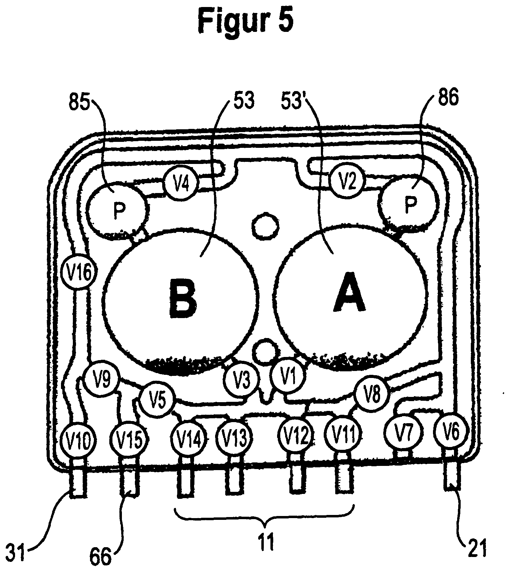

In Figur 5 ist ein zweites Ausführungsbeispiel für eine Kassette gezeigt. Die Kassette entspricht dabei in ihrer Ausführung im wesentlichen dem ersten Ausführungsbeispiel, umfasst jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z.B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluss 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so dass die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, dass sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, dass die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, dass die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfasst dabei eine Identifikationsmittel-Erfassungseinheit wie z. B. einen Barcodeleser oder eine RFID-Erfassungseinheit, welcher die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, dass die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

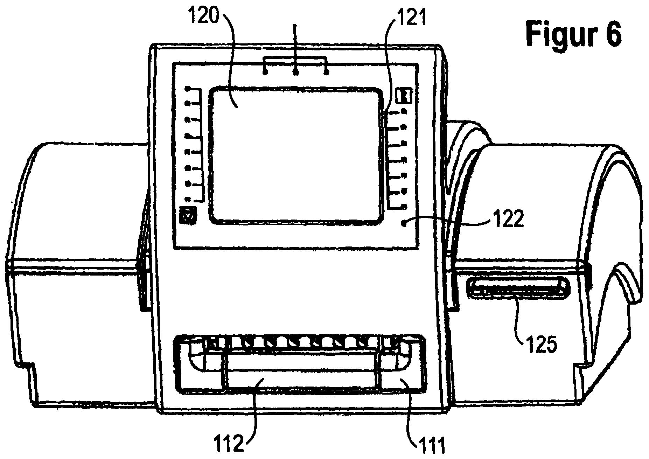

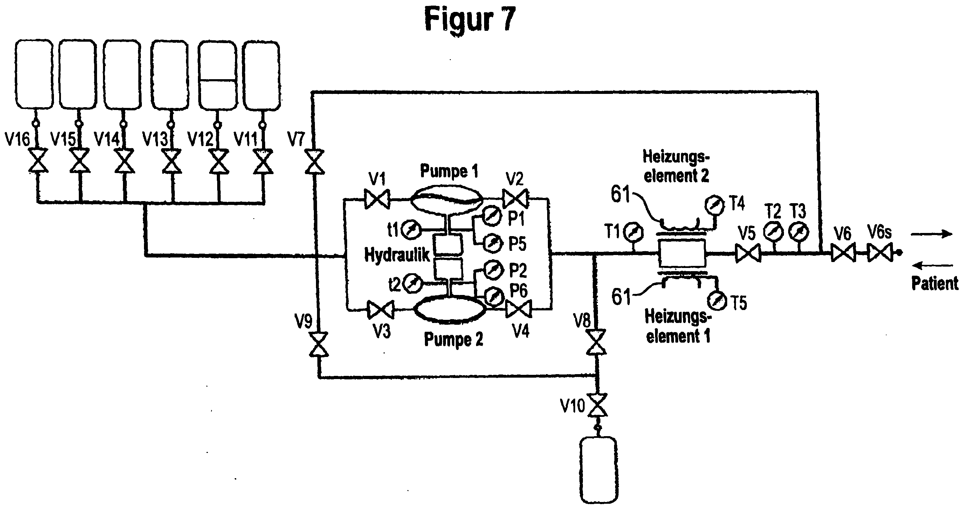

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

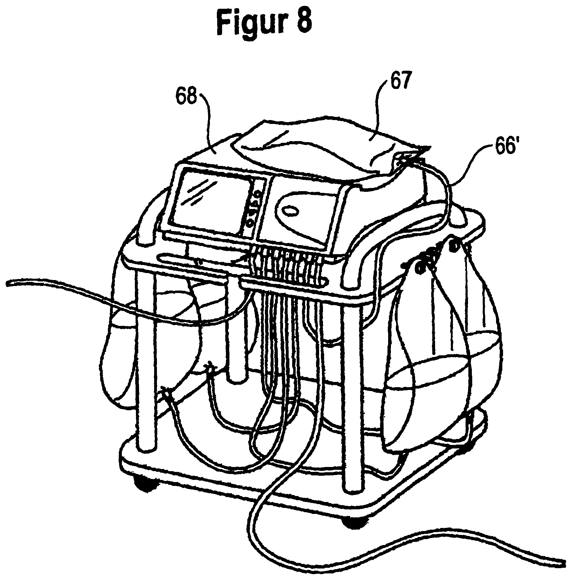

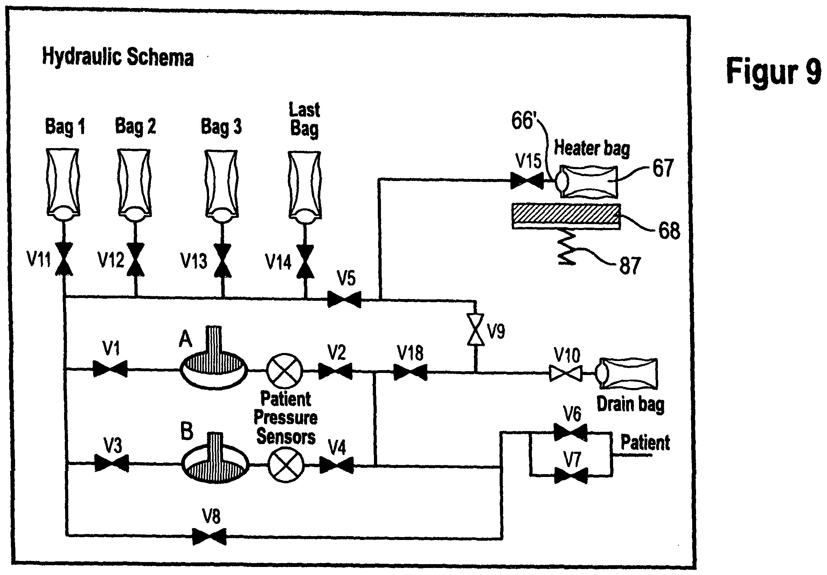

In Figur 8 ist ein zweites Ausführungsbeispiel einer Dialysemaschine gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muss auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so dass das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so dass sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so dass die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, dass die Heizplatte nicht zu heiß für das Material des Beutels wird.

Der Heizbeutel 67 kann zudem Funktionen bei der Billanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpresst, dass die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, dass die flexible Folie der Kassette mit den Stegbereichen der Kassette verpresst wird und so die Fluidwege innerhalb der Kassette voneinander trennt.

Vorteilhafterweise ist weiterhin ein umlaufender Rand der Ankoppelfläche vorgesehen, welcher mit dem Randbereich der Kassette verpresst wird. Vorteilhafterweise erfolgt die Verpressung dabei luftdicht, so dass zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelemente des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpresst.

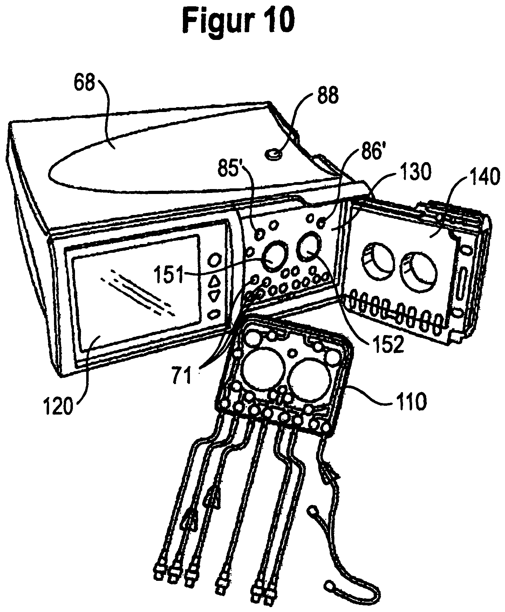

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so dass die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so dass eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug- und Pumphübe erfolgen, da die Membranpumpe eine hohe Genauigkeit der mit jedem Hub gepumpten Flüssigkeitsmenge aufweist.

### 2.3.1. Hydraulischer Antrieb

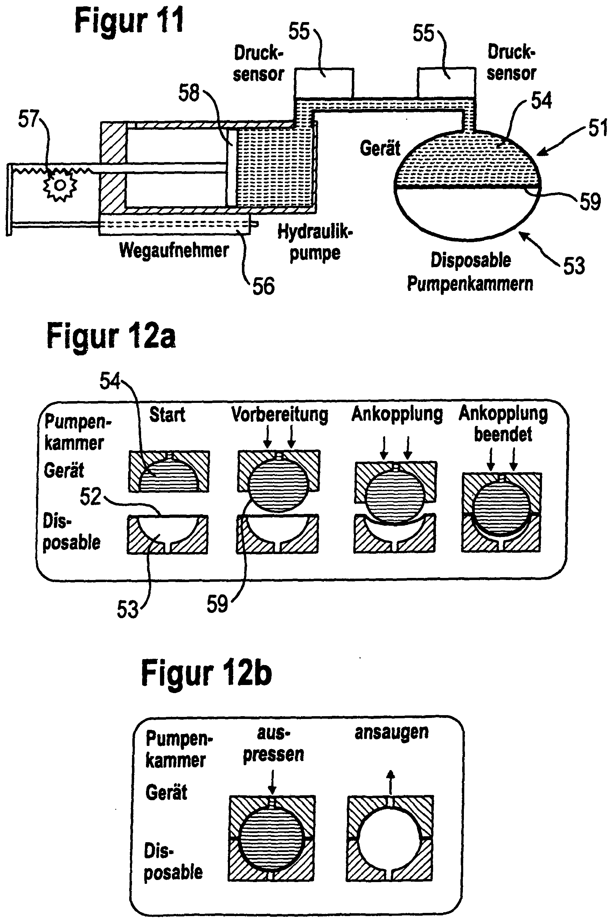

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so dass sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepreßt oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepreßt bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein.

Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppelns wird dabei zunächst die Kammer 54 mit Hydraulikfluid beaufschlagt, dass sich die Membran 59 nach außen wölbt. Daraufhin werden Ankoppelfläche und Kassette aufeinander zu bewegt, so dass die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so dass die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, dass die flexible Folie dem Stempel auch bei der Saugbewegung folgt.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so dass er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats mißt. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfasst weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialyse weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialyse weist weiterhin eine Steuerung auf, durch welche sämtliche Komponenten angesteuert und überwacht werden. Die Steuerung sorgt dabei für den automatischen Ablauf der Behandlung.

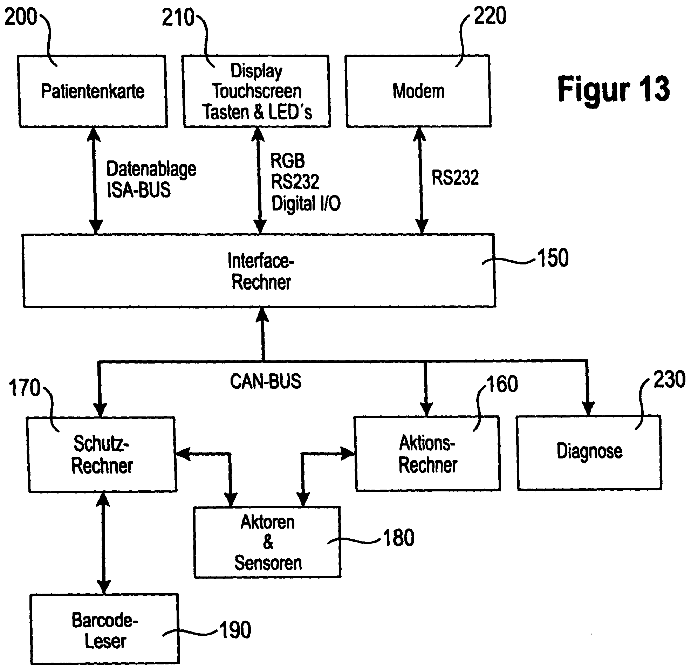

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so dass es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ein Ausführungsbeispiel der vorliegenden Erfindung, welches bei einem oben dargestellten Dialysesystemen bzw. bei einer der oben dargestellten Dialysemaschinen zum Einsatz kommen kann, wird nun im folgenden dargestellt. Dabei kann das Ausführungsbeispiel der vorliegenden Erfindung mit einzelnen oder mehreren Komponenten, wie sie oben beschrieben wurden, kombiniert werden. Insbesondere kann die Vorliegende Erfindung zur Ansteuerung einer Heizung eingesetzt werden, wie sie in Abschnitt 2.1. beschrieben wurde.

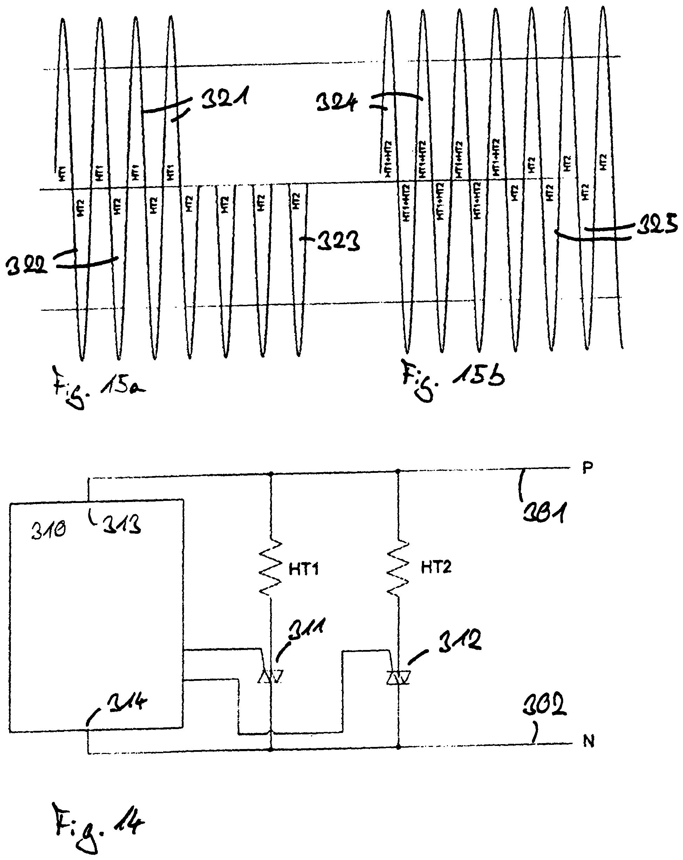

Fig. 14 zeigt dabei ein Ausführungsbeispiel einer erfindungsgemäßen Heizungssteuerung 310, durch welche die beiden Heizelemente HT1 und HT2 einer Heizung angesteuert werden. Die Heizungssteuerung umfaßt dabei ein erstes Schaltelement 311, durch welches das erste Heizelement HT1 ein- und ausgeschaltet werden kann, und ein zweites Schaltelement 312, mit welchem das zweiten Heizelement HT2 ein- und ausgeschaltet werden kann. Die beiden Heizelemente werden dabei durch die Schaltelemente 311 und 312 mit der an den Versorgungsleitungen 301 und 302 anliegenden Netzspannung beaufschlagt bzw. von dieser getrennnt. Die beiden Schaltelemente 311 und 312 werden dabei durch die Heizungssteuerung 310 angesteuert und bilden so eine Schaltanordnung. Bei den Schaltelementen 311 und 312 kann es sich beispielsweise um Triacs handeln. Die Netzspannung kann dabei ohne galvanische Trennung an den beiden Leitungen 301 und 302 anliegen. Alternativ kann die Netzspannung auch über eine galvanische Trennung an den Leitungen 301 und 302 anliegen, beispielsweise über einen Trenntransformator.

Weiterhin weist die Heizungssteuerung 310 Meßanschlüsse 313 und 314 zur Verbindung mit der Netzspannung auf. Dabei ist eine Überwachungsanordnung vorgesehen, welche die Nulldurchgänge der Netzspannung erkennt. Hierdurch kann die Schaltanordnung jeweils im Nulldurchgang der Netzspannung betätigt werden, um die Heizelemente ein- oder auszuschalten. Dabei wird erfindungsgemäß die Leistung der Heizung über das Ein- und Ausschalten einer oder mehrerer Halbwellen der Netzspannung angesteuert, insbesondere über das Verhältnis der Halbwellen mit eingeschalteten Heizelementen zur Anzahl der Halbwellen mit ausgeschalteten Heizelementen.
Bei dem in Fig. 14 gezeigten Ausführungsbeispiel sind dabei zwei Heizelemente vorgesehen, welche durch die Schaltanordnung unabhängig voneinander ein- und ausgeschaltet werden können. Hierdurch ergeben sich Vorteile, welche weiter unten noch näher beschrieben werden. Die vorliegende Erfindung kann jedoch auch mit einem Ausführungsbeispiel verwirklicht werden, bei welchem nur ein Heizelement HT1 oder HT2 vorgesehen ist. Durch das entsprechende Einstellen der Anzahl der Halbwellen mit eingeschaltetem Heizelement bzw. eingeschalteten Heizelementen kann damit eine Ansteuerung realisiert werden, welche es ermöglicht, zwischen 0 und 100% der Heizleistung zu realisieren. Insbesondere ist hierbei eine Temperaturregelung vorgesehen, bei welcher auf Grundlage eines Signals von einem Temperatursensor das Verhältnis der Anzahl der Halbwellen mit eingeschaltetem Heizelement zur Anzahl der Halbwellen mit ausgeschaltetem Heizelement eingestellt wird.
Weiterhin ermöglicht die vorliegende Erfindung, die Heizung mit unterschiedlichen Nennspannungen der Netzspannungsversorgung zu betreiben. Die Überwachungsanordnung der Heizungssteuerung 310 mißt hierfür die Höhe der Netzspannung und paßt die Ansteuerung des bzw. der Heizelemente entsprechend an die festgestellte Höhe der Netzversorgung an. Hierdurch kann eine gewünschte Leistung auch bei unterschiedlichen und/oder schwankenden Netzspannungen genau eingestellt werden, und es kann die gleiche Maximalleistung der Heizung bei unterschiedlichen Netzspannungen erreicht werden. In vorteilhafter Weise wird eine solche Anpassung an unterschiedlichen Netzspannungen dabei mit einer Temperaturregelung kombiniert.

Die im Ausführungsbeispiel in Fig. 14 gezeigte Verwendung von zwei unabhängig voneinander ansteuerbaren Heizelementen HT1 und HT2 ermöglicht dabei eine besonders günstige Anpassung an unterschiedliche Netzspannungen. Insbesondere können die beiden Heizelemente dabei in einem ersten Betriebsmodus gleichzeitig betrieben werden. Insbesondere können beide Heizelemente dabei synchron mit Netzspannungshalbwellen beaufschlagt werden. In diesem Betriebsmodus arbeiten die beiden Heizelemente daher im wesentlichen wie zwei parallel geschaltete Heizelemente mit nur einer Ansteuerung. Insbesondere kann ein solcher Betriebsmodus bei einer niedrigen Nennspannung von beispielsweise 100 V oder 120 V eingesetzt werden, um auch bei einer solchen niedrigen Netzspannung eine ausreichende maximale Heizleistung zur Verfügung zu stellen. Vorteilhafterweise schaltet die Steuerung dabei in den ersten Betriebsmodus, wenn sie eine Netzwechselspannung in einem ersten Spannungsbereich erkennt, welcher vorteilhafterweise Spannungen von 100V und 120V umfasst. In einem Ausführungsbeispiel kann sich der erste Spannungsbereich z.B. von 80V bis 160V erstrecken. Um die Heizleistung dabei auf einen gewünschten Wert einzustellen, können dabei die beiden Heizelemente selbstverständlich sowohl synchron ausgeschaltet werden, als auch abwechselnd, um das entsprechende Verhältnis der Anzahl der Halbwellen mit eingeschaltetem Heizelement zur Anzahl der Halbwellen mit ausgeschaltetem Heizelement auf den gewünschten Wert einzustellen.

Handelt es sich dabei um zwei Heizelemente mit identischer Nennleistung, so kann dabei die Anzahl der Halbwellen mit eingeschaltetem ersten Heizelement HT1 und die Anzahl der Halbwellen mit eingeschaltetem zweiten Heizelement HT2 zur Berechnung dieses Verhältnisses addiert werden. Ebenso kann die Anzahl der Halbwellen mit ausgeschalteten Heizelementen addiert werden. Haben die beiden Heizelemente dagegen unterschiedliche Nennleistungen, so muß dies durch einen entsprechenden Faktor berücksichtigt werden.

Im zweiten Betriebsmodus werden die beiden Heizelemente HT1 und HT2 dagegen jeweils abwechselnd mit Netzspannungshalbwellen beaufschlagt. Insbesondere wird dieser zweite Betriebsmodus dabei bei einer Nennspannung von 230 V oder 240 V eingesetzt. Vorteilhafterweise schaltet die Steuerung dabei in den zweiten Betriebsmodus, wenn sie eine Netzwechselspannung in einem zweiten Spannungsbereich erkennt, welcher höhere Spannungen umfasst als der erste Spannungsbereich. Vorteilhafterweise umfasst der zweite Spannungsbereich dabei Spannungen von 230V und 240V. In einem Ausführungsbeispiel kann sich der erste Spannungsbereich z.B. ab einer Spannung größer 160V erstrecken. Dadurch, daß im zweiten Betriebsmodus jeweils maximal eines der beiden Heizelemente mit Netzspannung beaufschlagt wird, kann die maximale Stromaufnahme unterhalb der zulässigen Stromstärke von beispielsweise 16 A gehalten werden. Um die maximale Heizleistung dabei auf einen gewünschten Wert einzustellen, bspw. auf die maximale Heizleistung im ersten Betriebsmodus, können dabei die beiden Heizelemente selbstverständlich auch beide ausgeschaltet werden, um das entsprechende Verhältnis der Anzahl der Halbwellen mit eingeschaltetem Heizelement zur Anzahl der Halbwellen mit ausgeschaltetem Heizelement auf den gewünschten Wert einzustellen.

In Fig. 15a und 15b sind dabei Ausführungsbeispiele für einen zweiten und einen ersten Betriebsmodus gezeigt. Dabei werden im zweiten Betriebsmodus in Fig. 15a jeweils einzelne Halbwellen abwechselnd auf das erste Heizelement HT1 und das zweite Heizelement HT2 geschaltet. Wie in Fig. 15a dargestellt, werden dabei die oberen Halbwellen 321 auf das erste Heizelement HT1 geschaltet, die unteren Halbwellen 322 auf das zweite Heizelement HT2. Selbstverständlich könnte die Umschaltung jedoch auch jeweils nach einer größeren Anzahl von Halbwellen erfolgen. In einem darauffolgenden Zeitabschnitt werden nur noch die unteren Halbwellen 323 auf das zweite Heizelement HT2 geschaltet, während die oberen Halbwellen komplett ausgeschaltet bleiben. Selbstverständlich könnten dabei auch einzelne Halbwellen so ausgeschaltet werden, daß zwischen dem abwechselnden Beaufschlagen des ersten Heizelements und des zweiten Heizelements jeweils Pausen liegen, in welchen Halbwellen komplett ausgeschaltet bleiben.

In Fig. 15b ist dagegen ein Ausführungsbeispiel des ersten Betriebsmodus gezeigt, in welchem sowohl die oberen Halbwellen 324 als auch die unteren Halbwellen 325 auf beide Heizelemente HT1 und HT2 geschaltet werden. Hierdurch kann eine entsprechend höhere Leistung erbracht werden.

In einem Ausführungsbeispiel trifft die Steuerung die Festlegung, ob eine Ansteuerung der Heizelemente HT1 und HT2 synchron (z.B. jeweils parallel mit Vollwellen) oder abwechselnd (z.B. jeweils mit Halbwellen) erfolgt, in Abhängigkeit von der detektierten Netzwechselspannung. Die Heizung ist dabei so ausgelegt, dass die volle Heizleistung bei einer minimalen Betriebsspannung (von z.B. 80V) und einem Tastgrad von 100% im Synchronbetrieb zur Verfügung gestellt werden kann. Ab einer vorbestimmten Grenzspannung (von z.B. 160V) werden die Heizelemente dagegen jeweils abwechselnd betrieben, wobei die Heizelemente z.B. abwechselnd separat mit einer Halbwelle (positiv bzw. negativ) angesteuert werden.

Eine Anpassung der Leistung an die Betriebsspannung oberhalb der minimalen Betriebsspannung erfolgt durch eine entsprechende Reduzierung der durchgelassenen Voll- bzw. Halbwellen, wobei im zweiten Betriebsmodus die Heizleistung ohnehin um 50% gegenüber dem Synchronbetrieb reduziert ist.

Bei den Heizelementen kann es sich dabei insbesondere um Ohmsche Heizelemente handeln. Diese können beispielsweise einen Widerstand zwischen 10 und 50 Ohm aufweisen. Die gewünschte Maximalheizleistung beträgt dabei beispielsweise zwischen 200 W und 2000 W, insbesondere ca. 800 W.

Im folgenden sollen nun zwei alternative Ausgestaltungen konkret beschrieben werden. Die maximale Sollheizleistung soll dabei jeweils 800 W betragen.

In einem ersten Ausführungsbeispiel werden dabei zwei Heizelemente mit einem Widerstand von 16 Ohm eingesetzt. Diese können bei einer Nennspannung von 110 V auch bei Berücksichtigung einer Unterspannung von 80 V im ersten Betriebsmodus die gewünschte Heizleistung von 800 W zur Verfügung stellen, wobei sich ein Strom von 10 A ergibt. Dabei werden beide Heizelemente mit der vollen Sinuswelle parallel angesteuert. Liegt die Spannung tatsächlich bei 110 V, so würde sich bei voller Ansteuerung beider Heizelemente eine Heizleistung von 1512 W ergeben. Daher werden entsprechend der gemessenen Spannung einzelne Halbwellen entweder an einem oder an beiden der Heizelemente abgeschaltet, um die maximale Heizleistung auf die gewünschten 800 W einzustellen. Bei 110 V Spannung werden daher ca. 52% aller Spannungshalbwellen tatsächlich eingeschaltet und die übrigen ausgeschaltet.

Bei einer Spannung von 240 V wird im zweiten Betriebsmodus gearbeitet, in dem immer maximal eines der beiden Heizelemente eingeschaltet ist. Bei einem Widerstand der Heizelemente von jeweils 16 Ohm ergibt sich dabei ein maximaler Stromfluß von 15 A.

Dabei würde sich bei einem Betrieb, bei welchem jede Halbwelle entweder auf das eine Heizelement oder das andere Heizelement geschaltet wird eine Heizleistung von 3600 W ergeben. Daher werden in Abhängigkeit von der Nennspannung entsprechend viele Halbwellen komplett unterdrückt, um die Heizleistung auf den gewünschten Maximalwert von 800 W einzustellen. Bei einer tatsächlichen Nennspannung von 240 V werden daher nur 22% aller Halbwellen entweder auf das eine oder das andere Heizelement geschaltet, so daß die gemittelte Stromaufnahme entsprechend sinkt. Es werden damit nur 11 % der im gleichphasigen Vollwellenbetrieb zu Verfügung stehenden Heizleistung bzw. Halbwellen genutzt.

Beim zweiten konkreten Ausführungsbeispiel werden zwei Heizelemente mit jeweils 25 Ohm eingesetzt. Bei einer effektiven Netzspannung von 100 V, wie sie zum Beispiel in Japan vorliegt, hat jeder der beiden Heizelemente damit eine maximale Heizleistung von 400 W. Im ersten Betriebsmodus, in welchem beide Heizelemente gleichphasig im Vollwellenbetrieb angesteuert werden, ergibt sich damit genau die gewünschte Heizleistung von 800 W.

Bei einer effektiven Netzspannung von 120 V, wie sie zum Beispiel in Amerika vorliegt, ergibt sich dagegen im gleichphasigen Vollwellenbetrieb eine Maximalleistung von 576 W für jedes der beiden Heizelemente. Um die Gesamtleistung bis auf 800 W zu reduzieren, wird daher die Anzahl der zur Erwärmung der Heizelemente eingesetzten Halbwellen der Netzspannung durch komplettes Ausschalten einzelner Halbwellen entsprechend reduziert. Dabei kann weiterhin gleichphasig gearbeitet werden und einzelne der Halbwellen komplett ausgeschaltet werden, oder einzelne Halbwellen nur für eines der beiden Heizelemente ausgeschaltet werden. Die Reduktion der Leistung auf die gewünschten 400 W ergibt sich dabei durch den Einsatz von nur noch 69% aller Halbwellen. Bezogen auf 255 Impulse werden daher nur 177 Impulse zum Heizen eingesetzt.

Bei einer Nennspannung von 240 V wird die Heizung dagegen im zweiten Betriebsmodus betrieben, in welchem das erste und das zweite Heizelement jeweils abwechselnd betrieben werden. Würde hier jede Halbwelle zum Betrieb eines der beiden Heizelemente eingesetzt, würde dies zu einer Heizleistung von ca. 2300 W führen. Um die Heizleistung auf die gewünschten 800 W zu reduzieren, muß daher ein entsprechender Anteil an Halbwellen komplett unterdrückt werden, so daß nur ca. 35% aller Halbwellen an einem der beiden Heizelemente eingesetzt werden, und damit nur ca. 17% der im gleichphasigen Vollwellenbetrieb zu Verfügung stehenden Heizleistung bzw. Halbwellen genutzt wird.

In vorteilhafter Weise werden die Halbwellen oder Pakete von Halbwellen dabei so geschaltet, daß am Heizelement keine Temperaturschwankung entsteht, das heißt das Schalten sollte schneller verlaufen als die Trägheit der Heizelemente.

Zum Beispiel kann die Ansteuerung dabei so erfolgen, daß das Verhältnis der Halbwellen mit eingeschaltetem Heizelement und mit ausgeschaltetem Heizelement über eine bestimmte Anzahl von Impulsen oder eine bestimmte Zeit gemittelt jeweils auf einem Sollwert gehalten wird. Beispielsweise kann dabei das Verhältnis über eine Periode von zum Beispiel 255 Halbwellen eingestellt werden.

Dabei ist die vorliegende Erfindung aber nicht darauf beschränkt, einzelne Halbwellen zu schalten. Vielmehr können ebenfalls Impulspakete mit mehreren Halbwellen geschaltet werden.

## Patentansprüche

1. Medizinisches Gerät mit einer Heizung mit mindestens einem Heizelement (HT1, HT2), welches von einer Heizungssteuerung (310) mit Netzspannung beaufschlagt wird, und mit einem Temperatursensor,
wobei die Heizungsteuerung (310) eine Überwachungsanordnung (313, 314) und eine Schaltanordnung (311, 312) umfasst, wobei die Überwachungsanordnung die Nulldurchgänge der Netzspannung erkennen und die Schaltanordnung das mindestens eine Heizelement im Nulldurchgang ein- oder auszuschalten kann,
**gekennzeichnet dadurch,**
**dass** die Heizungsteuerung (310) die Leistung der Heizung über das Ein- und Ausschalten einer oder mehrerer Halbwellen der Netzwechselspannung ansteuert, wobei das Verhältnis der Anzahl der Halbwellen mit eingeschalteten Heizelement zur Anzahl der Halbwellen mit ausgeschalteten Heizelement in Abhängigkeit von einem Signal des Temperatursensors eingestellt wird, wobei die Überwachungsanordnung (313, 314) weiterhin die Höhe der Netzwechselspannungsversorgung detektiert und die Heizungssteuerung (310) die Ansteuerung des mindestens einen Heizelementes (HT1, HT2) an die detektierte Höhe der Netzwechselspannungsversorgung anpasst, und zwar über das Verhältnis der Anzahl der Halbwellen mit eingeschaltetem Heizelement zur Anzahl der Halbwellen mit ausgeschaltetem Heizelement.

2. Medizinisches Gerät nach Anspruch 1, mit mindestens zwei Heizelementen (HT1, HT2), welche von der Schaltanordnung unabhängig voneinander ein- und ausgeschaltet werden können.

3. Medizinisches Gerät nach Anspruch 2, wobei die zwei Heizelemente (HT1, HT2) in einem ersten Betriebsmodus teilweise oder durchgehend synchron betrieben werden und insbesondere teilweise oder durchgehend synchron mit Netzwechselspannungshalbwellen beaufschlagt werden.

4. Medizinisches Gerät nach Anspruch 2 oder 3, wobei die zwei Heizelemente (HT1, HT2) in einem zweiten Betriebsmodus abwechselnd betrieben werden und insbesondere abwechselnd mit einer bestimmten Anzahl von Netzwechselspannungshalbwellen beaufschlagt werden.

5. Medizinisches Gerät nach Anspruch 3 und 4, wobei die Heizungsteuerung (310) in Abhängigkeit von der detektierten Höhe der Netzwechselspannungsversorgung den ersten oder zweiten Betriebsmodus auswählt, wobei vorteilhafterweise die Heizungsteuerung (310) bei Detektion einer Netzwechselspannung, welche sich in einem ersten, niedrigeren Spannungsbereich befindet, den ersten Betriebsmodus und bei Detektion einer Netzwechselspannung, welche sich in einem zweiten, höheren Spannungsbereich befindet, den zweiten Betriebsmodus auswählt.

6. Medizinisches Gerät nach einem der Ansprüche 3 bis 5, wobei bei einem Betrieb im ersten und/oder im zweiten Betriebsmodus das Verhältnis der Anzahl der Halbwellen mit eingeschalteten Heizelementen zur Anzahl der Halbwellen mit ausgeschalteten Heizelementen in Abhängigkeit von der Höhe der detektierten Netzwechselspannung eingestellt wird.

7. Medizinisches Gerät nach einem der vorangegangenen Ansprüche, wobei die Leistung der Heizung zu Zwecken der Temperaturregelung auf einen Wert unterhalb der Maximalleistung eingestellt wird, insbesondere auf einen Wert zwischen 0% und 100% der Maximalleistung.

8. Medizinisches Gerät nach einem der vorangegangenen Ansprüche, wobei die Heizungsteuerung (310) auf Grundlage des Signals des Temperatursensors ein Steuersignal erzeugt, welches den Steuersignalen zur Anpassung der Leistung an die detektierte Höhe der Netzwechselspannungsversorgung überlagert wird, wobei das Verhältnis der Anzahl der Halbwellen mit eingeschalteten Heizelementen zur Anzahl der Halbwellen mit ausgeschalteten Heizelement direkt in Abhängigkeit von dem Signal des Temperatursensors und der detektierten Höhe der Netzwechselspannungsversorgung zeitlich gleichmäßig eingestellt wird.

9. Medizinisches Gerät nach einem der vorangegangenen Ansprüche, wobei es sich bei dem medizinischen Gerät um ein Dialysegerät und bei der Heizung um eine Heizung (61) zur Erwärmung einer medizinischen Flüssigkeit handelt, wobei es sich vorteilhafterweise um ein Peritonealdialysegerät mit einer Heizung zur Erwärmung des Dialysats handelt.

10. Medizinisches Gerät nach einem der vorangegangenen Ansprüche, wobei als Temperatursensor ein Temperatursensor eingesetzt wird, welcher die Temperatur des Heizelementes (HT1, HT2) direkt misst und/oder ein Temperatursensor, welcher die Temperatur des zu erwärmenden Mediums bestimmt, insbesondere die Temperatur des Dialysats beim Einsatz in einem Peritonealdialysegerät.

11. Medizinisches Gerät nach einem der vorangegangenen Ansprüche, wobei die zur Ansteuerung verwendete kleinste Anzahl von schaltbaren Halbwellen bei 1 bis 5 Halbwellen liegt, vorteilhafterweise bei 1 bis 3 Halbwellen.

12. Verfahren zum Betrieb eines medizinischen Gerätes mit einer Heizung mit mindestens einem Heizelement (HT1, HT2) und mit einem Temperatursensor oder zum Betrieb einer Heizungssteuerung (310) für ein solches Gerät, mit den Schritten
Erfassen der Nulldurchgänge der Netzspannung und
Ein- oder Ausschalten des mindestens einen Heizelements (HT1, HT2) im Nulldurchgang, wobei die Leistung der Heizung über die Anzahl der Halbwellen der Netzwechselspannung mit eingeschaltetem Heizelement angesteuert wird, wobei das Verhältnis der Anzahl der Halbwellen mit eingeschalteten Heizelement zur Anzahl der Halbwellen mit ausgeschalteten Heizelement in Abhängigkeit von einem Signal des Temperatursensors eingestellt wird, wobei weiterhin die Höhe der Netzwechselspannungsversorgung detektiert wird und die Heizungssteuerung (310) die Ansteuerung des mindestens einen Heizelementes (HT1, HT2) an die detektierte Höhe der Netzwechselspannungsversorgung anpasst, und zwar über das Verhältnis der Anzahl der Halbwellen mit eingeschaltetem Heizelement zur Anzahl der Halbwellen mit ausgeschaltetem Heizelement.

13. Verfahren nach Anspruch 12 zum Betrieb eines medizinischen Gerätes oder einer Heizungssteuerung nach einem der vorangegangenen Ansprüchen.

## Claims

1. A medical device having a heater with at least one heating element (HT1, HT2) that is acted on with mains voltage by a heating control (310) and having a temperature sensor,
wherein the heating control (310) comprises a monitoring arrangement (313, 314) and a switching arrangement (311, 312), wherein the monitoring arrangement can recognize the zero points of the mains voltage and the switching arrangement can switch at least one heating element on or off at the zero point,
**characterized in that**
the heating control (310) controls the power of the heater by the switching on and off of one or more half-cycles of the AC mains voltage, with the ratio of the number of half-cycles with a heating element switched on to the number of half-cycles with the heating element switched off being set in dependence on a signal of the temperature sensor, wherein the monitoring arrangement (313, 314) furthermore detects the level of the AC mains supply and the heating control (310) adapts the control of the at least one heating element (HT1, HT2) to the detected level of the AC mains supply, and this via the ratio of the number of the half cycles with a switched on heating element to the number of the half cycles with a switched off heating element.

2. A medical device in accordance with claim 1, having at least two heating elements (HT1, HT2) which can be switched on or off independently of one another by the switching arrangement.

3. A medical device in accordance with claim 2, wherein the two heating elements (HT1, HT2) are operated partly or continuously synchronously in a first operating mode and are in particular acted on partly or continuously synchronously with AC mains voltage half-cycles.

4. A medical device in accordance with claim 2 or claim 3, wherein the two heating elements (HT1, HT2) are alternately operated in a second operating mode and are in particular acted on alternately by a specific number of AC mains voltage half-cycles.

5. A medical device in accordance with claim 3 or claim 4, wherein the heating control (310) selects the first or second operating mode in dependence on the detected level of the AC mains voltage supply, wherein the heating control (310) advantageously selects the first operating mode on a detection of an AC mains voltage which is in a first, lower voltage range and selects the second operating mode on a detection of an AC main voltage which is in a second, higher voltage range.

6. A medical device in accordance with one of the claims 3 to 5, wherein, on an operation in the first and/or second operating modes, the ratio of the number of the half-cycles with switched on heating elements to the number of the half-cycles with switched off heating elements is set in dependence on the level of the detected AC mains voltage.

7. A medical device in accordance with one of the preceding claims, wherein the power of the heater is set to a value below the maximum power, in particular to a value between 0% and 100% of the maximum power, for the purposes of temperature control.

8. A medical device in accordance with one of the preceding claims, wherein the heating control (310) generates a control signal on the basis of the signal of the temperature sensor, said control signal being superposed on the control signals for adapting the power to the detected level of the AC mains voltage supply; and wherein the ratio of the number of half-cycles with heating elements switched on to the number of half-cycles with heating elements switched off is set uniformly in time directly in dependence on the signal of the temperature sensor and on the detected level of the AC mains voltage supply.

9. A medical device in accordance with one of the preceding claims, wherein the medical device is a dialysis machine and the heater is a heater (61) for heating a medical fluid; and wherein it is advantageously a peritoneal dialysis machine having a heater for heating the dialyzate.

10. A medical device in accordance with one of the preceding claims, wherein a temperature sensor is used as the temperature sensor that directly measures the temperature of the heating element (HT1, HT2) and/or a temperature sensor that determines the temperature of the medium to be heated, in particular the temperature of the dialyzate on a use of a peritoneal dialysis machine.

11. A medical device in accordance with one of the preceding claims, wherein the smallest number of switchable half-cycles for the control is 1 to 5 half-cycles, advantageously 1 to 3 half-cycles.

12. A method of operating a medical device having a heater with at least one heating element (HT1, HT2) and having a temperature sensor or for operating a heating control (310) for such a device, said method comprising the steps of
detecting the zero points of the mains voltage; and
switching the at least one heating element (HT1, HT2) on or off at the zero point, wherein the power of the heater is controlled by the number of half-cycles of the AC mains voltage with a heating element switched on, with the ratio of the number of half-cycles with a heating element switched on to the number of half-cycles with a heating element switched off is set in dependence on a signal of the temperature sensor, wherein furthermore the level of the AC mains supply is detected and the heating control (310) adapts the control of the at least one heating element (HT1, HT2) to the detected level of the AC mains supply, and this via the ratio of the number of the half cycles with a switched on heating element to the number of the half cycles with a switched off heating element.

13. A method in accordance with claim 12 for operating a medical device or a heating control in accordance with one of the preceding claims.

## Revendications

1. Appareil médical équipé d'un chauffage, comprenant au moins un élément chauffant (HT1, HT2), qui est alimenté en tension réseau par une commande de chauffage (310), et un capteur de température,
la commande de chauffage (310) comprenant un ensemble de surveillance (313, 314) et un ensemble de commutation (311, 312), l'ensemble de surveillance pouvant reconnaître les passages par zéro de la tension réseau et l'ensemble de commutation pouvant mettre en marche ou arrêter l'au moins un élément chauffant lors du passage par zéro,
**caractérisé en ce que**
la commande de chauffage (310) commande la puissance du chauffage par le biais de l'activation et de la désactivation d'une ou de plusieurs demi-ondes de la tension alternative du réseau, le rapport du nombre des demi-ondes avec élément chauffant en marche au nombre des demi-ondes avec élément chauffant arrêté étant réglé en fonction d'un signal du capteur de température,
dans lequel
l'ensemble de surveillance (313, 314) détecte en outre la hauteur de l'alimentation en tension alternative du réseau et la commande de chauffage (310) adapte la commande de l'au moins un élément chauffant (HT1, HT2) à la hauteur détectée de l'alimentation en tension alternative du réseau, et cela par le biais du rapport du nombre des demi-ondes avec élément chauffant en marche au nombre des demi-ondes avec élément chauffant arrêté.

2. Appareil médical selon la revendication 1, comprenant au moins deux éléments chauffants (HT1, HT2), qui peuvent être mis en marche et arrêtés indépendamment l'un de l'autre par l'ensemble de commutation.

3. Appareil médical selon la revendication 2, dans lequel,
dans un premier mode de fonctionnement, les deux éléments chauffants (HT1, HT2) sont exploités en partie ou en continu de manière synchrone et sont en particulier alimentés en demi-ondes de tension alternative du réseau en partie ou en continu de manière synchrone.

4. Appareil médical selon la revendication 2 ou 3, dans lequel,
dans un second mode de fonctionnement, les deux éléments chauffants (HT1, HT2) sont exploités alternativement et sont en particulier alimentés alternativement avec un nombre déterminé de demi-ondes de tension alternative du réseau.

5. Appareil médical selon les revendications 3 et 4, dans lequel
la commande de chauffage (310) sélectionne le premier ou le second mode de fonctionnement en fonction de la hauteur détectée de l'alimentation en tension alternative du réseau, la commande de chauffage (310) sélectionnant de manière avantageuse le premier mode de fonctionnement lors de la détection d'une tension alternative du réseau qui se trouve dans une première plage de tension plus basse, et le second mode de fonctionnement lors de la détection d'une tension alternative du réseau qui se trouve dans une seconde plage de tension plus élevée.

6. Appareil médical selon l'une des revendications 3 à 5, dans lequel,
lors d'un fonctionnement dans le premier et/ou dans le second mode de fonctionnement, le rapport du nombre des demi-ondes avec éléments chauffants en marche au nombre des demi-ondes avec éléments chauffants arrêtés est réglé en fonction de la hauteur de la tension alternative du réseau détectée.

7. Appareil médical selon l'une des revendications précédentes, dans lequel
la puissance du chauffage est réglée à des fins de régulation de température sur une valeur inférieure à la puissance maximale, en particulier sur une valeur entre 0 % et 100 % de la puissance maximale.

8. Appareil médical selon l'une des revendications précédentes, dans lequel
la commande de chauffage (310) génère, sur la base du signal du capteur de température, un signal de commande qui est superposé aux signaux de commande pour l'adaptation de la puissance à la hauteur détectée de l'alimentation en tension alternative du réseau, le rapport du nombre des demi-ondes avec éléments chauffants en marche au nombre des demi-ondes avec éléments chauffants arrêtés étant réglé directement en fonction du signal du capteur de température et de la hauteur détectée de l'alimentation en tension alternative du réseau, de manière régulière dans le temps.

9. Appareil médical selon l'une des revendications précédentes, dans lequel l'appareil médical est un appareil de dialyse et le chauffage est un chauffage (61) destiné à chauffer un liquide médical, dans lequel il s'agit de manière avantageuse d'un appareil de dialyse péritonéale équipé d'un chauffage destiné à chauffer le dialysat.

10. Appareil médical selon l'une des revendications précédentes, dans lequel on utilise comme capteur de température un capteur de température qui mesure directement la température de l'élément chauffant (HT1, HT2) et/ou un capteur de température qui détermine la température du milieu à chauffer, en particulier la température du dialysat lors de l'utilisation dans un appareil de dialyse péritonéale.

11. Appareil médical selon l'une des revendications précédentes, dans lequel le nombre le plus faible de demi-ondes commutables utilisé pour la commande est de 1 à 5 demi-ondes, de manière avantageuse de 1 à 3 demi-ondes.

12. Procédé de fonctionnement d'un appareil médical équipé d'un chauffage, comprenant au moins un élément chauffant (HT1, HT2) et un capteur de température ou de fonctionnement d'une commande de chauffage (310) pour un appareil de ce type, comprenant les étapes
détection des passages par zéro de la tension réseau et
mise en marche ou arrêt de l'au moins un élément chauffant (HT1, HT2) lors du passage par zéro, la puissance du chauffage étant commandée par le biais du nombre des demi-ondes de la tension alternative du réseau avec élément chauffant en marche, le rapport du nombre des demi-ondes avec élément chauffant en marche au nombre des demi-ondes avec élément chauffant arrêté étant réglé en fonction d'un signal du capteur de température, et en outre la hauteur de l'alimentation en tension alternative du réseau est détecte et la commande de chauffage (310) adapte la commande de l'au moins un élément chauffant (HT1, HT2) à la hauteur détectée de l'alimentation en tension alternative du réseau, et cela par le biais du rapport du nombre des demi-ondes avec élément chauffant en marche au nombre des demi-ondes avec élément chauffant arrêté.

13. Procédé selon la revendication 12 pour le fonctionnement d'un appareil médical ou d'une commande de chauffage selon l'une des revendications précédentes.
